(19) Europäisches Patentamt
European Patent Office
Office européen des brevets

(11) Veröffentlichungsnummer: **0 030 585**
B1

(12) # EUROPÄISCHE PATENTSCHRIFT

(45) Veröffentlichungstag der Patentschrift: **27.07.83**

(51) Int. Cl.³: **C 07 C 51/367**, C 07 C 59/68, C 07 D 213/64 // A01N39/02

(21) Anmeldenummer: **80103214.5**

(22) Anmeldetag: **10.06.80**

(54) **Verfahren zur Herstellung von Phenoxipropionsäuren und ihren Alkalisalzen.**

(30) Priorität: **11.12.79 DE 2949728**

(43) Veröffentlichungstag der Anmeldung: **24.06.81 Patentblatt 81/25**

(45) Bekanntmachung des Hinweises auf die Patenterteilung: **27.07.83 Patentblatt 83/30**

(84) Benannte Vertragsstaaten: **BE CH DE FR GB IT LI NL**

(56) Entgegenhaltungen:
**DE-A-2 136 828**
**DE-A-2 531 643**
**GB-A-1 422 679**
**US-A-3 113 965**
**US-A-3 493 604**

(73) Patentinhaber: **HOECHST AKTIENGESELLSCHAFT, Postfach 80 03 20, D-6230 Frankfurt/Main 80 (DE)**

(72) Erfinder: **Koch, Manfred, Dr., Kreuzheck 2, D-6239 Eppstein/Taunus (DE)**
Erfinder: **Nestler, Hans Jürgen, Dr., Steinweg 15, D-6240 Königstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 030 585 B1

BUNDESDRUCKEREI BERLIN

Verfahren zur Herstellung von Phenoxipropionsäuren und ihren Alkalisalzen

Es ist bekannt, daß alkyl- und/oder halogen-substituierte Phenoxiessig-, bzw. -propionsäuren, welche als sogenannte Wuchsstoffherbizide (im folgenden Wuchsstoffsäuren genannt) von großer wirtschaftlicher Bedeutung sind, durch Umsetzung von Alkaliphenolaten mit den Alkalisalzen der entsprechenden 2-Halogenalkancarbonsäuren hergestellt werden können.

Im allgemeinen wird die Umsetzung so durchgeführt, daß Alkaliphenolat und das Alkalisalz der 2-Halogenalkancarbonsäure im Molverhältnis 1 : 1 in Wasser als Lösungs- bzw. Verdünnungsmittel kondensiert werden (siehe G. Erfurt et. al., Chem. Technik 15 (1964), Nr. 4, Seite 199; DD-PSs 64 279, 64 972, 64 723).

Wegen der dabei nicht vermeidbaren Nebenreaktion (Hydrolyse von Halogenalkancarbonsäuresalz zu Hydroxyalkancarbonsäuresalz) ist die Ausbeute nicht befriedigend (70—85% d. Th.). Auch wird die Ausbeute beim Übergang von 2-Chloressigsäure zu höheren 2-Chloralkancarbonsäuren wie 2-Chlorpropionsäure und 2-Chlorbuttersäure erheblich vermindert (DE-PS 11 53 762).

Die Ausbeute an Wuchsstoffsäuren kann durch Zurückdrängung der Hydrolyse erhöht werden. Bekanntgeworden sind folgende Möglichkeiten:

a) Verwendung von wasserfreien Reaktanten (US-PS 26 51 659). Obwohl hier wesentlich bessere Ausbeuten an Wuchsstoffsäuren erzielt werden, ist diese Variante für eine technische Durchführung wenig geeignet, da

   — die Herstellung von wasserfreien Agentien, insbesondere der hydrolyseempfindlichen Alkalisalze der 2-Halogenalkancarbonsäuren schwierig und teuer ist,
   — große Volumina organischer Lösungsmittel notwendig sind,
   — Feststoffe oder Suspensionen dosiert werden müssen,
   — als unerwünschte Nebenreaktion in größerem Umfang Lactate, durch Alkylierung der Phenoxialkancarbonsäuresalze mit Halogenalkancarbonsäuresalz, entstehen.

b) Teilweiser Ersatz von Wasser als Lösungs- und Verdünnungsmittel durch höher siedende Alkohole (DE-PS 1 153 762, US-P-2 914 558, SU-PS 187 766, Jap. Anm., Nr. 48 705/65), durch Kohlenwasserstoffe (US-PS 2 480 817) oder durch überschüssiges freies Phenol (US-PS 4 035 496, DD-PS 50 622.
c) Anwendung eines Überschusses bis zu 100% an Alkaliphenolat bezogen auf eingesetztes 2-Halogenalkancarbonsäuresalz zur Erhöhung der Geschwindigkeit der Hauptreaktion gegenüber der Hydrolyse-Nebenreaktion (US-PS 4 035 416, DE-PS 1 153 762, JA-AS-74/24 463, US-PS-3 257 453).
d) Reduzierung der im Reaktionsgemisch vorhandenen Wassermenge durch Abdestillieren von Wasser während der Umsetzung. Bei der in US-PS 4 035 416 beschriebenen Versuchsanordnung wird Natronlauge mit überschüssigem 2,4-Dichlorphenol versetzt und ein Teil (ca. 35%) des im Reaktionsgemisch vorhandenen Wassers abdestilliert, anschließend werden äquimolekulare Mengen an Natronlauge und Chloressigsäure, im Unterschuß bezogen auf Natriumdichlorphenolat simultan zudosiert, wobei gleichzeitig Wasser abdestilliert wird.

Es ist weiterhin bekanntgeworden, daß optisch aktive Wuchsstoffsäuren (DE-AS 1 543 841) durch Kondensation von alkyl- und/oder halogen-substituierten Alkaliphenolaten und Alkalisalzen der L-2-Chlorpropionsäure in Kohlenwasserstoffen wie z. B. Toluol hergestellt werden; d. h. entsprechend der unter b) genannten Methodik. Die Ausbeute an optisch aktiver Wuchsstoffsäure beträgt bei diesem Verfahren 85—90% d. Th. Grundsätzlich gemeinsam ist allen bekannten Verfahren zur Herstellung von Wuchsstoffsäuren:

— Die zur Umsetzung vorgesehene Phenolmenge wird als Alkaliphenolat vorgelegt, dazu wird 2-Halogenalkancarbonsäuresalz in einer Portion, in mehreren Portionen oder kontinuierlich zugesetzt. In manchen Fällen wird die insgesamt notwendige Alkalimenge als überschüssiges Alkaliphenolat oder als Mischung von Alkaliphenolat und Alkalihydroxid vorgelegt und 2-Halogenalkancarbonsäure zudosiert.
  Die Phenolkomponente wird nicht quantitativ umgesetzt. Nach erfolgter Umsetzung ist eine Abtrennung und Rückgewinnung von nicht umgesetztem Phenol notwendig.

Eine analoge Herstellung von Phenoxypropionsäuren der Formel I ist nach oben genannten Verfahren nicht möglich, da es nur durch aufwendige und langwierige Reinigungsoperationen unter Verlust gelingt, die als Ausgangsmaterialien verwendeten Phenole der Formel II von den Verbindungen der Formel I abzutrennen.

Zwar sind für die Herstellung dieser Verbindungen gleichfalls schon eine Anzahl von Verfahren bekanntgeworden, die aber auch nicht zu quantitativen Ausbeuten führen:

In der DE-OS 16 68 896 wird neben anderen Herstellungsmethoden die Umsetzung von α-Halogenfettsäuren mit Phenoxiphenolen in wäßriger, stark alkalischer, Lösung beschrieben. Verfährt man nach dieser, an den früheren Stand der Wuchsstoffsäurenherstellung angelehnten Verfahrensweise, indem man Phenoxiphenole mit einem Überschuß von 20% L-2-Chlorpropionsäure in wäßriger stark alkalischer Lösung kondensiert, erhält man nur einen Umsatz von 82% d. Th. Ein Umsetzungsgrad >95% der Phenolkomponente wird erst bei wesentlich höherem Überschuß an L-2-Chlorpropionsäure (>40%) erreicht. Ein solches Verfahren ist zur quantitativen Umsetzung der Phenole der Formel II in technischem Maßstab nicht geeignet.

Die Verbesserung dieser Umsetzung durch Ersatz von Wasser als Lösungs- bzw. Verdünnungsmittel durch einen Kohlenwasserstoff wie Toluol oder Xylol und azeotrope Abdestillierung von Wasser während der Umsetzung (s. Herstellung Wuchsstoffsäuren) führt ebenfalls nicht zu einer quantitativen Umsetzung der Phenole der Formel II. So wird mit einem Überschuß von 20% der 2-Chlorpropionsäure (bezogen auf eingesetztes Phenol) nur ein Umsatz von 92—93% der Phenolkomponente erzielt.

Es wurde nun gefunden, daß die Nachteile der beschriebenen Verfahren vermieden werden können, wenn man anstelle der Alkaliphenolate die freien Phenole einsetzt (wodurch ein konstant hoher Entwässerungsgrad erzielt wird) und außerdem dafür sorgt, daß sich stets nur soviel Alkali-Chlorpropionat in der Reaktionsmischung befindet, wie mit dem (durch gleichzeitige Zugabe von Alkalihydroxid gebildeten) Alkaliphenolat reagieren kann. Dadurch wird ein Überschuß von Alkalihydroxid während des Ablaufs der Hauptreaktion im Reaktionsgemisch, der eine teilweise Hydrolyse der 2-Chlorpropionsäure zu Milchsäure zur Folge hätte, stark zurückgedrängt.

Gegenstand der vorliegenden Erfindung ist somit ein neues halbkontinuierliches Verfahren zur Herstellung von Phenoxipropionsäuren und ihren Alkalisalzen der allgemeinen Formel I:

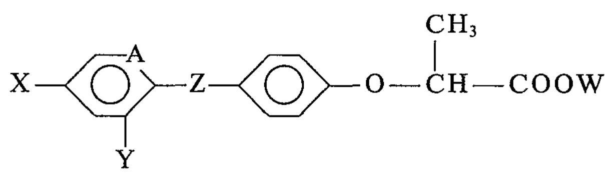
(I)

in welcher W = Wasserstoff oder ein Alkalikation darstellt und Z für Sauerstoff oder $CH_2$ steht, wobei im Falle

Z = O:
X = Cl, Br oder $CF_3$: A = —N= oder —CH= und
Y = H oder Cl

und im Falle

Z = $CH_2$:
X = Cl; A = —CH= und Y = H oder Cl

bedeuten, sowie von deren optisch aktiven D-Isomeren, das dadurch gekennzeichnet ist, daß man Verbindungen der Formel

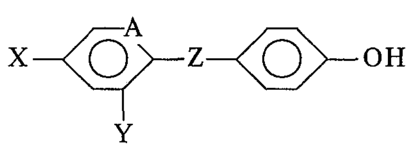
(II)

in einem mit Wasser Azeotrope bildenden Lösungsmittel bei Siedetemperatur und Abdestillieren des im Verlauf der Reaktion eingebrachten oder entstehenden Wassers mit 2-Chlorpropionsäure oder 2-Chlorpropionsäure-niederalkylester oder deren D-Isomere unter gleichzeitiger Zugabe der doppelt molaren Menge (bezogen auf die Chlorpropionsäurekomponente) oder eines geringen Überschusses an wäßrigem Alkalihydroxid umsetzt.

2-Chlorpropionsäure und Alkalilauge (vorzugsweise verwendet man 30—50%ige Natronlauge) können entweder getrennt zudosiert oder vor der Zugabe zu II vereinigt werden, wobei quantitativ Alkali-2-chlorpropionat gebildet wird. Man kann das Alkali-2-chlorpropionat auch durch Verseifung von 2-Chlorpropionsäureniederalkylester mit überschüssigem Alkalihydroxid herstellen und die Reaktionslösung wie beschrieben mit den Phenolen der Formel II umsetzen. Schließlich kann man auch 2-Chlorpropionsäureester und Alkalihydroxid getrennt aber gleichzeitig zudosieren. In diesem Falle erfolgt in situ zunächst die Verseifung des Esters zu 2-Chlorpropionsäure und dann deren Umsetzung mit II.:

Man verwendet pro Mol II die 1,0 bis 1,4-molare Menge an 2-Chlorpropionsäure(ester). Für eine

quantitative Umsetzung sollte ein Überschuß von mindestens 10%, vorzugsweise von 12—25% angewendet werden.

Zweckmäßig wird die Umsetzung mit der Mindestmenge an Alkalihydroxid oder einem geringen Überschuß (bis zu 10%) durchgeführt, doch führen auch höhere Überschüsse nicht zu einer signifikanten Verminderung der Ausbeute. Als Alkalihydroxid verwendet man vorzugsweise NaOH; auch KOH kommt in Betracht.

Als Lösungs- bzw. Verdünnungsmittel sind mit Wasser azeotrop destillierende Kohlenwasserstoffe geeignet. Bevorzugt werden aromatische Kohlenwasserstoffe wie Benzol, Toluol, Xylol oder Chlorbenzol verwendet, am vorteilhaftesten sind Xylol und Toluol.

Man verfährt bei der Reaktion dergestalt, daß man die Verbindung der Formel II im Lösungsmittel löst, wobei die Menge des Lösungs- oder Verdünnungsmittels so gewählt wird, daß die Reaktionsmischung bei Reaktionsende noch ausreichend rührbar ist (im allgemeinen werden etwa 0,3—3 Liter pro Mol verwendet).

Die Zugabe von 2-Chlorpropionsäure(ester) und Alkalilauge (simultan und getrennt) bzw. der wäßrigen Lösung von Natrium-2-chlorpropionat und Alkalihydroxid erfolgt so, daß das in die Reaktionsmischung eingebrachte oder durch Salzbildung entstandene Wasser stets azeotrop abdestilliert wird. Die im Reaktionsgemisch vorhandene Wassermenge soll vorzugsweise 2,5%, bezogen auf das Gewicht der eingesetzten Reaktanten, nicht überschreiten.

Gemäß einer Variante der erfindungsgemäßen Verfahrens gibt man zu der annähernd auf Siedetemperatur erhitzten Lösung des Phenols aus zwei getrennten Dosiergefäßen annähernd gleichzeitig unter kräftigem Rühren die 1,0 bis 1,4fache Moläquivalentmenge des 2-Chlorpropionsäureesters — bevorzugt ist der Methylester — sowie die 2,0 bis 2,8fache Moläquivalentmenge einer 20 bis 60%igen, bevorzugt 25 bis 50%igen, wäßrigen Alkalihydroxidlösung. Zulaufgeschwindigkeit und Reaktionstemperatur werden so geregelt, daß sich die Wasserzufuhr zuzüglich der Reaktionswasserbildung und die Wasserabscheidung durch Azeotropdestillation etwa entsprechen. Zusammen mit dem Wasser destilliert der aus dem Ester durch Verseifung entstandene Alkohol zum größten Teil ab. Eine exakt gleichzeitige Zugabe der beiden Komponenten ist nicht unbedingt erforderlich.

Während der Reaktion ist die Temperatur zweckmäßig zwischen dem Siedepunkt des reinen Lösungsmittels als Obergrenze und demjenigen, der sich durch die Anwesenheit der verfahrensmäßig bedingten geringen Wassermenge im Reaktionsgemisch einstellt, als Untergrenze zu halten. Sie beträgt z. B. bei Verwendung von Xylol etwa 106—115°C. Zur Minimierung der zur azeotropen Abtrennung benötigten Lösungsmitteldampfmenge wird die Umsetzung bevorzugt an der unteren Temperaturgrenze durchgeführt. Für Benzol beträgt die Reaktionstemperatur bevorzugt 74—80°C, für Toluol bevorzugt 100—110°C.

Nach Vollendung der Zugabe wird zur Vervollständigung der Umsetzung bei gleichbleibender Reaktionstemperatur etwa 15 bis 30 min nachgerührt, anschließend können die entstandenen Natriumsalze der Phenoxipropionsäuren durch Ansäuern mit Mineralsäure in die freien Säuren überführt werden. Die im Reaktionsgemisch anwesenden anorganischen Salze und Milchsäure werden durch Ausrühren mit Wasser abgetrennt.

Nach dem Abdestillieren des Lösungsmittels erhält man die Verfahrenserzeugnisse der allgemeinen Formel I (W=H) in einer Ausbeute von 99,5—99,9% d. Th. Der Restgehalt an Ausgangsmaterial der allgemeinen Formel II beträgt <0,5%.

Bei Verwendung von optisch reiner L-2-Chlorpropionsäure, bzw. eines Niedrigalkylesters liegt die optische Reinheit der so hergestellten D-(Phenoxi- bzw. Benzyl-)-phenoxipropionsäuren zwischen 86 und 92% d. Th., entsprechend einem Gehalt von 93—96% an reinen D-Isomeren.

Die Verbindungen der Formel I sind selbst hochwirksame Gräserherbizide oder Vorprodukte für andere Säurederivate, wie zum Beispiel Ester, welcher ebenfalls hochwirksame, selektive Gräserherbizide darstellen (DE-OS 2 223 894, 2 433 067, 2 601 548, 2 417 487 und 2 758 002. Durch folgende Beispiele wird das erfindungsgemäße Verfahren erläutert:

## Beispiele

### Beispiel 1

2-[4′-(2″,4″-Dichlorphenoxy)-phenoxy]-propionsäure

127,2 Gew.-Teile 2-Chlorpropionsäure (98%ig und 80 Gew.-Teile Wasser (oder Eis) werden unter äußerer Kühlung so mit 118 Gew.-Teilen 50%iger Natronlauge versetzt, daß die Innentemperatur 40°C nicht übersteigt. Die erhaltene viskose Flüssigkeit dosiert man bei 115°C zu einer Lösung von 225 Gew.-Teilen 4-(2′,4′,-Dichlorphenoxy)-phenol in 900 Gew.-Teilen Toluol. Die Reaktionstemperatur wird während der Zugabe durch azeotropes Abdestillieren von Wasser am Wasserabscheider bei 115°C gehalten. Nach beendeter Zugabe wird 15 min bei 105°C nachgerührt, auf 90°C abgekühlt und nach Zugabe von 400 Teilen Wasser 20 min bei 85°C gerührt, anschließend wird bei 85°C mit 120 Gew.-Teilen Phosphorsäure angesäuert, 10 min gerührt, anschließend die Wasserphase abgetrennt.

Toluol wird im Wasserstrahlvakuum abdestilliert und der erhaltene Feststoff bei 80° C und 250 mbar getrocknet. Man erhält 327 Gew.-Teile 2-[4'-(2",4"-Dichlorphenoxy)-phenoxy]-propionsäure, der Gehalt beträgt laut Gaschromatogramm 99,5% entsprechend einer Ausbeute von 99,5% d. Th. Der Restgehalt an 4-(2',4'-Dichlorphenoxy)-phenol beträgt 0,4%.

Beispiel 2

2-[4'-(4"-Trifluormethylphenoxy)-phenoxy]-propionsäure

Zu einer Lösung von 254 Gew.-Teilen 4-(4'-Trifluormethylphenoxy)-phenol in 1 000 Gew.-Teilen Xylol wird bei 110—115° C exakt simultan, jedoch getrennt 132,5 Gew.-Teile 2-Chlorpropionsäure (98%ig) und 199 Gew.-Teile 50%ige Natronlauge zudosiert. Die Reaktionstemperatur wird während der Zugabe durch azeotropes Abdestillieren von Wasser am Wasserabscheider eingehalten. Nach beendeter Zugabe wird 15 min bei 110—115° C nachgerührt, auf 90° C abgekühlt und nach Zugabe von 400 Teilen Wasser bei 85° C 10 min gerührt, anschließend wird bei 85° C mit 120 Teilen Phosphorsäure angesäuert und 10 min bei dieser Temperatur gerührt. Nach Abtrennung der Wasserphase wird Xylol am Wasserstrahlvakuum abdestilliert und der Feststoff bei 60° C, 250 mbar getrocknet. Man erhält 326 Gew.-Teile 2-[4'-(4"-Trifluormethylphenoxy)-phenoxy]-propionsäure. Der Gehalt an reinem Endprodukt beträgt 99,6% entsprechend einer Ausbeute von 99,6% d. Th. Der Restgehalt an 4-(4'-Trifluormethylphenoxy)-phenol beträgt 0,35%.

Beispiel 3

2-[4'-(4"-chlorbenzyl)-phenoxy]-propionsäure

129,8 Gew.-Teile 2-Chlorpropionsäure, 194 Gew.-Teile 50%ige Natronlauge und 80 Gew.-Teile Wasser werden wie in Beispiel 1 beschrieben vermischt.

Die so erhaltene klare, viskose Flüssigkeit wird zu einer Lösung von 218,5 Gew.-Teilen 4-(4'-Chlorbenzyl)-phenol in 1 000 Gew -Teilen Xylol zudosiert, wobei die Reaktionstemperatur durch azeotropes Abdestillieren von Wasser am Wasserabschneider zwischen 110° C und 115° C gehalten wird. Nach beendeter Zugabe wird 15 min bei 110° C nachgerührt. Die Aufarbeitung erfolgt wie in Beispiel 2 beschrieben. Man erhält 290,5 Gew.-Teile 2-[4'-(4"-Chlorbenzyl)-phenoxy]-propionsäure. Der Gehalt an reinem Endprodukt beträgt 99,8% entsprechend einer Ausbeute von 99,8% d. Th. Der Restgehalt an 4-(4'-Chlorbenzyl)-phenol beträgt 0,1%.

Analog wurden hergestellt

| | Ausbeute [% d. Th.] | Restgehalt Ausgangsmaterial |
|---|---|---|
| 2-[4'-(4"-Chlorphenoxy)-phenoxy]-propionsäure | 99,8% | 0,1% |
| 2-[4'-(4"-Brom-2"-chlorphenoxy)-phenoxy]-propionsäure | 99,6% | 0,3% |
| 2-[4'-(2",4"-Dichlorbenzyl)-phenoxy]-propionsäure | 99,7% | 0,1% |
| 2-[4'-(2"-Chlor-4"-trifluormethyl-phenoxy)-phenoxy]-propionsäure | 99,7% | 0,2% |

Beispiel 4

D(+)-2-[4'-(2",4"-Dichlorphenoxy)-phenoxy]-propionsäure

34,3 g (0,28 mol) L(−)-2-Chlorpropionsäuremethylester ($\alpha_D^{23} = -24,7°$, in Substanz) und 30 ml Wasser werden bei 15° C mit 45,7 g (0,57 mol) Natronlauge (50%ig) versetzt und nach Zugabe von 30 Minuten bei 15° C nachgerührt. Die entstandene klare Lösung wird zu einer Lösung von 63,8 g (0,25 mol) 4-(2',4'-Dichlorphenoxy)-phenol in 300 ml Xylol bei 110—115° C zudosiert. Während der

Zugabe wird durch kontinuierliches azeotropes Abdestillieren von Wasser und Methanol eine Innentemperatur von 110—115°C eingehalten. Nach Zugabe wird 30 Minuten bei 115°C nachgerührt, anschließend bei 90°C 100 ml Wasser zugesetzt, weitere 30 Minuten bei 85°C gerührt, schließlich mit 25 g o-Phosphorsäure angesäuert und die Wasserphase im Scheidetrichter abgetrennt. Nach Abdestillieren des Xylols erhält man 81,7 g D(+)-2-[4'-(2'',4''-Dichlorphenoxy)-phenoxy]-propionsäure. Laut Gaschromatogramm sind 99,5% der gewünschten Säure und 0,5% unumgesetztes Phenol enthalten, entsprechend einer Ausbeute von 99,5% d. Th. Die erhaltene Säure wird mit Methanol unter Katalyse von Schwefelsäure verestert. Das Drehvermögen des (D+)-Methyl-2-[4'-(2'',4''-Dichlorphenoxy)-phenoxy]-propionats beträgt $[\alpha]_D^{25}$+25,7° (l-molare Lösung in $CHCl_3$), entsprechend einer optischen Reinheit von 94%.

## Beispiel 5

### D(+)-2-[4'-(2'',4''-Dichlorphenoxy)-phenoxy]-propionsäure

30,2 g 49%ige Natronlauge (0,37 mol Natriumhydroxid) und 20 g Eis werden bei 15°C mit 19,8 g L(−)-2-Chlorpropionsäure (0,18 mol, 99%ige; gem. Drehwert—15,9°) versetzt. Die erhaltene klare Lösung wird zu einer Lösung von 38,3 g (0,15 mol) 4-2',4'-Dichlorphenoxy)-phenol in 300 ml Toluol bei 105°C unter kontinuierlichem azeotropem Abdestillieren von Wasser zudosiert. Nach Zugabe wird 30 Minuten bei 105°C nachgerührt, anschließend bei 85°C 70 ml Wasser zugesetzt, weitere 30 Minuten bei 80°C gerührt, schließlich mit 15 g o-Phosphorsäure angesäuert und die Wasserphase abgetrennt. Nach Abdestillieren des Toluols erhält man 49 g D(+)-2-[4'-(2'',4''-Dichlorphenoxy)-phenoxy]-propionsäure mit einer gaschromatisch bestimmten Reinheit von 99,9%. Der Restgehalt an 4-(2',4'-Dichlorphenoxy)-phenol beträgt 0,1%, entsprechend einer Ausbeute von 99,9% d. Th. Die erhaltene Säure wird wie in Beispiel 1 beschrieben mit Methanol verestert. Das Drehvermögen des D(+)-Methyl-2-[4'-(2'',4''-Dichlorphenoxy)-phenoxy]-propionats beträgt $[\alpha]_D^{25}$ =+24,1° (1-molare Lösung in $CHCl_3$), entsprechend einer optischen Reinheit von 88%.

## Beispiel 6

### D(+)-2-[4'-(2'',4''-Dichlorphenoxy)-phenoxy]-propionsäure

Zu einer Lösung von 63,8 g (0,25 mol) 4-[2',4'-Dichlorphenoxy)-phenol in 300 ml Xylol werden bei 110—115°C getrennt und exakt simultan 32,9 g L(−)-2-Chlorpropionsäure (99%ig, Drehwert gemessen ohne Lösungsmittel −15,9°, 23°C) und 49,6 g 50%ige Natronlauge (0,62 mol Natriumhydroxid) zudosiert, wobei die Reaktionstemperatur durch kontinuierliches azeotropes Abdestillieren von Wasser eingehalten wird. Nach Zugabe wird 30 Minuten bei 115°C nachgerührt, anschließend bei 90°C 100 ml Wasser zugesetzt, weitere 25 g o-Phosphorsäure angesäuert und die Wasserphase im Scheidetrichter abgetrennt. Nach Abdestillieren des Xylols erhält man 81,5 g D(+)-2-[4'-(2'',4''-Dichlorphenoxy)-phenoxy]-propionsäure. Der Restgehalt an unumgesetzter Phenolkomponente beträgt 0,2%, entsprechend einer Ausbeute von 99,7% d. Th. Der Drehwert des daraus durch Versterung mit Methanol erhaltenen D(+)-Methyl-2-[2'',4''-Dichlorphenoxy)-phenoxy]-propionats beträgt 23,7° $[\alpha]_D^{23}$ =23,7° (1-molare Lösung in $CHCl_3$).

## Beispiel 7

### D(+)-2-[4'-(2'',4''-Dichlorphenoxy)-phenoxy]-propionsäure

36,8 g (0,3 mol) L(−)-2-Chlorpropionsäuremethylester ($\alpha_D^{23}$ = −23,9°, in Substanz) und 40 ml Wasser werden bei 15°C mit 49,9 g (0,62 mol) Natronlauge (50%ig) versetzt und nach Zugabe 30 Minuten bei 15°C nachgerührt. Die entstandene klare Lösung wird zu einer Lösung von 55,1 g (0,25 mol) 4-(2',4'-Dichlorphenoxy)-phenol in 300 ml Xylol bei 110—115°C zudosiert. Während der Zugabe wird durch kontinuierliches azeotropes Abdestillieren von Wasser und Methanol eine Innentemperatur von 110—115°C eingehalten. Nach Zugabe wird 30 Minuten bei 115°C nachgerührt, anschließend bei 90°C 100 ml Wasser zugesetzt, weitere 30 Minuten bei 85°C gerührt, schließlich mit 25 g o-Phosphorsäure angesäuert und die Wasserphase im Scheidetrichter abgetrennt. Nach Abdestillieren des Xylols erhält man 73,1 g D(+)-2-[4'-(2'',4''-Dichlorphenoxy)-phenoxy]-propionsäure. Laut Gaschromatogramm sind 99,9% der gewünschten Säure und 0,1% ungesetztes Phenol enthalten, entsprechend einer Ausbeute von 99,9% d. Th. Die erhaltene Säure wird mit Methanol unter Katalyse von Schwefelsäure verestert. Das Drehvermögen des D(+)-Methyl-2-[4'-(2'',4''-Dichlorphenoxy)-phenoxy]-propionats beträgt $[\alpha]_D^{25}$ =24,4° (1-molare Lösung in $CHCl_3$).

Analog wurden hergestellt

| Verbindung | Ausbeute | Restgehalt Ausg. | Opt. Drehung des Methylesters (1-molar in $CHCl_3$) | |
|---|---|---|---|---|
| | [% d. Th.] | Phenol | $[\alpha]_D$ | t [°C] |
| D(+)-2-[4'-(2'',4''-Dichlorbenzyl)-phenoxy]-propionsäure | 99,7 | 0,2 | 26,5° | 23 |
| D(+)-2-[4'-(4''-Trifluormethylphenoxy)-phenoxy]-propionsäure | 99,9 | 0,1 | + 25,2° | 23 |
| D(+)-2-[4'-(4''-Trifluormethyl-2''-chlor-phenoxy]-propionsäure | 99,7 | 0,2 | – | – |
| D(+)-2-[4'-(4''-Brom-2''-chlorphenoxy)-phenoxy]-propionsäure | 99,8 | 0,15 | + 23,1° | 23 |

Beispiel 8

D-2-[4'-(2'',4''-Dichlorphenoxy)-phenoxy]-propionsäure

Zu einer Lösung von 255 g (1,0 mol) 4-(2',4'-Dichlorphenoxy)-phenol in 1000 ml Xylol, die auf 110°C vorgeheizt wurde, gibt man unter kräftigem Rühren aus 2 Tropftrichtern gleichzeitig insgesamt 150 g (1,2 mol) L-2-Chlorpropionsäuremethylester (opt. Reinheit > 95%) sowie insgesamt 200 g (2,5 mol) 50 gew.-%ige Natronlauge im Laufe von ca. einer Stunde zu. Über einen Wasserabscheider wird simultan zur Zugabe das Gemisch von entstandenem Methanol und zugeführtem bzw. gebildetem Wasser durch azeotrope Destillation abgenommen. Man läßt ca. 30 min. nachreagieren, kühlt ab und setzt aus dem kristallin ausgefallenen Natriumsalz die D-2-[4'-(Dichlor-phenoxy)-phenoxy]-propionsäure durch Ansäuern mit verdünnter Schwefelsäure in Freiheit. Man erhält nach Abtrennen der wäßrigen Phase und Abdampfen des Lösungsmittels 320 g = 98% d. Th. an Rohprodukt.

Chemische und optische Reinheit wurden nach Überführung in den Methylester ermittelt. Der so erhaltene D-2-[4-(2'',4''-Dichlorphenoxy)-phenoxy]-propionsäuremethylester hat eine chemische Reinheit von 95% (GC). Der optische Drehwert ist $[\alpha]_D^{23}$ = 22,0° (1 m in Chloroform), entsprechend einer optischen Reinheit von 79%.

**Patentanspruch**

Verfahren zur Herstellung von Phenoxidpropionsäuren und ihren Alkalisalzen der allgemeinen Formel

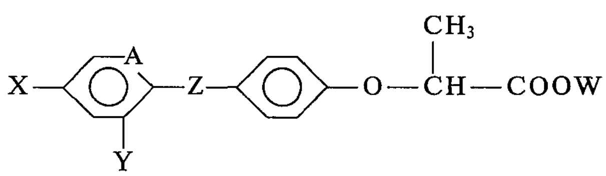

(I)

in welcher W = Wasserstoff oder ein Alkalikation darstellt und Z für Sauerstoff oder $CH_2$ steht, wobei im Falle Z = O

X = Cl, Br oder $CF_3$; A = –N= oder –CH= und
Y = H oder Cl

und im Falle Z = $CH_2$

X = Cl; A = –CH= und
Y = H oder Cl

bedeuten, sowie von deren optisch aktiven D-Isomeren, dadurch gekennzeichnet, daß man

Verbindungen der Formel

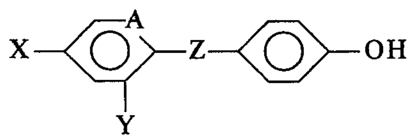

(II)

in einem mit Wasser Azeotrope bildenden Lösungsmittel bei Siedetemperatur und Abdestillieren des im Verlauf der Reaktion eingebrachten oder entstehenden Wassers mit 2-Chlorpropionsäure oder 2-Chlorpropionsäureniederalkylester oder deren D-Isomere unter gleichzeitiger Zugabe der doppelt molaren Menge (bezogen auf die Chlorpropionsäurekomponente) oder eines geringen Überschusses an wäßrigem Alkalihydroxid umsetzt.

## Claim

A process for the preparation of phenoxypropionic acids and the alkali metal salts thereof corresponding to the general formula

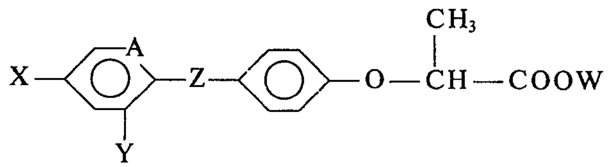

(I)

in which
W is hydrogen or an alkali metal cation, and Z is oxygen or $CH_2$, provided that in the case where Z is O, X is Cl, Br or $CF_3$, A is $-N=$ or $-CH=$ and Y is H or Cl; and in the case where Z is $CH_2$, X is Cl, A is $-CH=$ and Y is H or Cl, and their optically active D-isomers, which comprises reacting compounds of the formula II

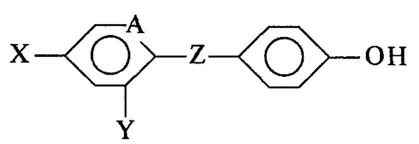

(II)

with 2-chloropropionic acid or 2-chloropropionic acid lower alkyl ester or the D-isomers thereof in a solvent forming an azeotropic mixture with water and at reflux temperature while simultaneously adding the double molar amount, relative to the chloropropionic acid component or a slight excess thereover, of aqueous alkali hydroxide and distilling off the water introduced or formed during the reaction.

## Revendication

Procédé de préparation d'acides phénoxypropioniques et de leurs sels de métaux alcalins qui répondent à la formule générale I:

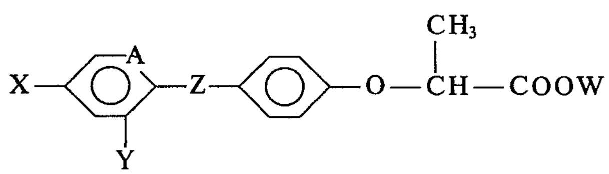

(I)

dans laquelle

W représente l'hydrogène ou un cation de métal alcalin,
Z représente l'oxygène ou $CH_2$,

dans le cas où Z représente O:

X représente Cl, Br ou $CF_3$,
A représente $-N=$ ou $-CH=$ et
Y représente H ou Cl,

et dans le cas où Z représente $CH_2$:

X représente Cl,
A représente $-CH=$ et
Y représente H ou Cl

ainsi que de leurs isomères optiquement actifs D, procédé caractérisé en ce qu'on fait réagir des composés de formule II

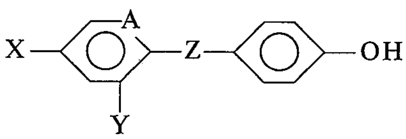

(II)

dans un solvant capable de former un azétorope avec l'eau, à la température d'ébullition et en chassant par distillation l'eau introduite ou engendrée au cours de la réaction, avec l'acide chloro-2 propionique ou un ester alkylique inférieur de l'acide chloro-2 propionique ou leurs isomères D, en même temps qu'on ajoute une quantité double de la quantité molaire (par rapport à la composante acide chloropropionique) ou un léger excès d'hydroxyde de métal alcalin aqueux.